# EUROPEAN PATENT APPLICATION

(11) **EP 2 929 786 A1**
(43) Date of publication of application: **14.10.2015**
(21) Application number: 13859937.8
(22) Date of filing: 22.07.2013
(51) Int. Cl.: A23L 1/29, A23L 1/30, A23L 2/38

(54) **COMPOSITION COMPRISING NATURAL POLYPHENOL COMPOUNDS, AND COMPOSITION FOR ORAL ADMINISTRATION COMPRISING SAME**

(30) Priority: 05.12.2012 KR 20120140548
(71) Applicant: Samsung Fine Chemicals Co., Ltd., Ulsan-city 680-090 (KR)
(72) Inventor: KO, Won Hwa, Incheon 403-816 (KR); BAEK, Hyon Ho, Incheon 406-722 (KR); HONG, Jun Kee, Yongin-si Gyeonggi-do 448-728 (KR); LEE, Sung Wan, Incheon 402-200 (KR); CHA, Ja Hyun, Incheon 400-070 (KR)
(74) Representative: Kador & Partner
(86) International application number: PCT/KR2013/006523
(87) International publication number: WO 2014/088182

(57) **Abstract**

Disclosed are a composition containing a natural polyphenolic compound and an orally ingestible composition including the composition containing a natural polyphenolic compound. The composition containing a natural polyphenolic compound may include a poorly water-soluble natural polyphenolic compound (PSNPPC) and saponin.

## Description

### TECHNICAL FIELD

The inventive concept relates to a composition containing a natural polyphenolic compound and a orally ingestible composition, and more particularly, to a composition containing a natural polyphenolic compound having excellent water dispersibility or water solubility and a orally ingestible composition including the composition containing a natural polyphenolic compound.

### BACKGROUND ART

The global market for functional food offering health benefits has continuously grown at an annual average rate of 6% in the last 5 years due to the spread of global well-being trends and population aging. It seems that the growth of this market will further increase as the number of health-oriented customers has constantly increased. In Korea too the market for functional food has rapidly increased so that the importance of the market has gradually increased.

Furthermore, although single functional products have conventionally represented the mainstream, patterns have recently changed, and thus the market for the multi-functional products has taken over from the market for single functional products market and various functional healthy foods have appeared. Such functional healthy foods include many natural products found in nature. Although the majority of natural products have a variety of beneficial effects, such as a strong anti-oxidation function, they also have low water solubility, and thus, the bioavailability thereof upon oral ingestion decreases.

### DETAILED DESCRIPTION OF THE INVENTIVE CONCEPT

### TECHNICAL PROBLEM

The inventive concept provides a composition containing a natural polyphenolic compound having excellent water dispersibility or water solubility.

The inventive concept provides an orally ingestible composition including the composition containing a natural polyphenolic compound.

### TECHNICAL SOLUTION

According to an aspect of the inventive concept, there is provided a composition containing a natural polyphenolic compound including a poorly water-soluble natural polyphenolic compound (PSNPPC) and saponin, and having water dispersibility or water solubility.

The PSNPPC may have a solubility of 0.004 to 0.087.

The PSNPPC may include curcumin, silymarin, resveratrol, or a combination thereof.

The saponin may include tea saponin, American ginseng saponin, Panax ginseng saponin (leaf), Panax ginseng saponin (root), soy saponin, or a combination thereof.

When dissolving the composition, a solubility of the PSNPPC may be in a range of 0.006 to 1.087.

An amount of the saponin may be in a range of 25 to 400 parts by weight based on 100 parts by weight of the PSNPPC.

According to another aspect of the inventive concept, there is provided an orally ingestible composition including the composition containing a natural polyphenolic compound.

The orally ingestible composition may be a functional healthy food or a medicine in a form of a tablet, a capsule, a liquid formulation, or a beverage.

An amount of the saponin in the orally ingestible composition may be in a range of 0.4 to 50 wt%.

### ADVANTAGEOUS EFFECTS

The composition containing a natural polyphenolic compound according to an exemplary embodiment has excellent water dispersibility or water solubility, and thus, when being used for oral ingestion, the bioavailabilty thereof is high.

### BEST MODE

Hereinafter, a composition containing a natural polyphenolic compound according to an exemplary embodiment will be described in detail.

According to an aspect of one or more exemplary embodiments, there is provided a composition containing a natural polyphenolic compound includes a poorly water-soluble natural polyphenolic compound (PSNPPC) and saponin and have water dispersibility or water solubility. In the present specification, "water dispersibility" refers to a nature in which the composition containing a PSNPPC exists in an emulsion state in water, and "water solubility" refers to a nature in which the composition containing a natural polyphenolic compound exists in a solution state in water.

The PSNPPC may have a solubility of 0.004 to 0.087. In the present specification. "solubility" refers to an amount (mg) of a solute (PSNPPC) that dissolves in 1mL of water at 37°C, which is similar to a human body temperature.

The PSNPPC may have a physiological function.

The PSNPPC may include curcumin, silymarin, resveratrol, or a combination thereof. The curcumin may be derived from an extract of a turmeric root, and the silymarin may be derived from an extract of milk thistle, and the resveratrol may be derived from an extract of grapes or the like.

The saponin may have a physiological function, such as an immunopotentiating function and a metabolism promoting function, and furthermore, increase water dispersibility or water solubility of the PSNPPC.

The saponin may include tea saponin, American ginseng saponin, Panax ginseng saponin (leaf), Panax ginseng saponin (root), soy saponin, or a combination thereof.

When dissolving the composition containing a natural polyphenolic compound, a solubility of the PSNPPC may be in a range of 0.006 to 1.087. When a solubility of the PSNPPC is within this range, the bioavailability of the PSNPPC may increase. In the present specification, "solubility of PSNPPC, when dissolving a composition containing a natural polyphenolic compound" means an amount (mg) of PSNPPC that is dissolved in water from among components of the composition containing a natural polyphenolic compound, when adding the composition containing a natural polyphenolic compound to 1 mL of water at 37°C.

An amount of the saponin may be in a range of 25 to 400 parts by weight based on 100 parts by weight of the PSNPPC. When an amount of the saponin is within this range, water dispersibility and water solubility of the PSNPPC increases, thereby improving bioavailability thereof.

The composition containing a natural polyphenolic compound configured as described above according to an exemplary embodiment may intactly maintain its active ingredients when dispersed or dissolved in water, while the PSNPPC thereof is not physicochemically changed, and may be used for various purposes, as a method of preparation is simple.

According to another aspect of one or more exemplary embodiments, there is provided an orally ingestible composition including the composition containing a natural polyphenolic compound.

The orally ingestible composition may be a functional healthy food or a medicine in a form of a tablet, a capsule, liquid formulation, or beverage. In the present specification, "liquid formulation" means a liquid preparation that may be in a typical liquid form or show consistency, and is distributed while filled in a container in a form of ampoules or vials having a capacity of 10 to 30 mL, and "beverage" means a thing that is in a typical drink form and distributed while filled in a container having a capacity of 75 to 120 mL.

The tablet may be prepared by using a conventional preparation method, such as a direct compression. In particular, the tablet may be prepared by mixing the orally ingestible composition homogeneously, and then compressing the resultant by using a tablet molding compressor.

The capsule may be prepared by mixing the orally ingestible composition homogeneously, and then, filling a hard capsule with the resultant.

The liquid formulation and beverage may be prepared by adding the orally ingestible composition to purified water, which confers a unique smell and flavor to the composition, and the resultant may be filled in a container and packed to allow easy drinking.

An amount of the saponin included in the orally ingestible composition may be in a range of 0.4 to 50 wt%. When an amount of the saponin is within this range, bioavailability of the PSNPPC may increase, and an orally ingestible composition providing an excellent physiological function may be obtained.

### MODE OF THE INVENTIVE CONCEPT

Hereinafter, the inventive concept will be described in detail with reference to Examples; however, the inventive concept is not limited thereto.

### Example

### Experimental Example 1: Solubility of poorly water-soluble natural polyphenolic compound (PSNPPC)

Solubility of 3 types of poorly water-soluble natural polyphenolic compounds (PSNPPC) (a product of Naturex SA of France) were measured, and the results thereof are shown in Table 1.

**[Table 1]**

| | Curcumin Silymarin | Resveratrol |
|---|---|---|
| Solubility (@37°C) | 0.004 0.008 | 0.087 |

### Experimental Example 2: Solubility of PSNPPC when adding additives

In 3 types of PSNPPCs, 11 types of additives were mixed in a ratio of 1:0.25 (624 mg:156 mg), 1:1 (625 mg:625 mg), or 1:4 (625 mg:2500 mg) as described in Table 2 below, to prepare a powder mixture, and then, the mixture was added to 45 mL of water and stirred. Afterward, 5 mL of the resultant was taken, and was filtrated and diluted so as to use as a test liquid for high-performance liquid chromatography (HPLC). That is, constituents and an amount of each constituent of the test liquid were quantified by using the HPLC. Then, solubility of each compound with respect to 1 mL of water was calculated by using an amount of PSNPPC that had been detected by using HPLC. In addition, the solubility measurement results are shown in Table 2. The sources of the additives listed in Table 2 below are as follows: tea saponin (Hunan Amazing Grace biotechnology Co., Ltd., China), American ginseng saponin (Ningbo Dekang Biochem Co., Ltd., China), Panax ginseng saponin (leaf) (Ningbo Dekang Biochem Co., Ltd., China), Panax ginseng saponin (root) (Hong-ziu Biotech Co., Ltd., China), soy saponin (Xi'an Greenherb Biotech Co., Ltd., China), PEG 400 (Samchun pure chemical, Korea), Tween 80 (Sigma Aldrich, U.S.A), β-Aescin amorphors (Nuri PharmTech Co., Ltd., Korea), Hawthorn Fruit P.E. (Shannxi M.R Natural product Co., Ltd., China), Quillaja saponaria saponin (Xi'an sinuote Bio-tech Co., Ltd., China), and Tribulus terrestris saponin (Ningbo Tianhong Biotech Co., Ltd., China).

**[Table 2]**

| Additive | | Solubility of PSNPPC (@37°C) | | |
|---|---|---|---|---|
| Type | Addition ratio (PSNPPC:additive) (Based on weight) | Curcumin | Silymarin | Resveratrol |
| Tea saponin | 1:0.25 | 0.008 | 0.194 | 0.104 |
| | 1:1 | 0.057 | 0.540 | 0.273 |
| | 1:4 | 0.182 | 1.087 | 0.866 |
| American ginseng | 1:0.25 | 0.012 | 0.168 | 0.112 |
| saponin | 1:1 | 0.060 | 0.347 | 0.312 |
| | 1:4 | 0.174 | 0.728 | 0.980 |
| Panax ginseng saponin (leaf) | 1:0.25 | 0.007 | 0.083 | 0.105 |
| | 1:1 | 0.045 | 0.180 | 0.287 |
| | 1:4 | 0.168 | 0.435 | 1.024 |
| Panax ginseng saponin (root) | 1:0.25 | 0.006 | 0.085 | 0.105 |
| | 1:1 | 0.035 | 0.213 | 0.312 |
| | 1:4 | 0.127 | 0.531 | 1.020 |
| Soy saponin | 1:0.25 | 0.007 | 0.030 | 0.026 |
| | 1:1 | 0.043 | 0.091 | 0.090 |
| | 1:4 | 0.118 | 0.325 | 0.320 |
| PEG 400 | 1:0.25 | 0.000 | 0.060 | 0.039 |
| | 1:1 | 0.000 | 0.083 | 0.111 |
| | 1:4 | 0.002 | 0.124 | 0.212 |
| Tween^{®} 80 | 1:0.25 | 0.054 | 0.143 | 0.289 |
| | 1:1 | 0.049 | 0.419 | 0.658 |
| | 1:4 | 0.639 | 1.436 | 1.015 |
| β-Aescin amorphors (root) | 1:0.25 | 0.000 | 0.072 | 0.005 |
| | 1:1 | 0.000 | 0.124 | 0.194 |
| | 1:4 | 0.000 | 0.219 | 0.030 |
| Hawthorn Fruit P.E. | 1:0.25 | 0.000 | 0.111 | 0.062 |
| | 1:1 | 0.001 | 0.183 | 0.076 |
| | 1:4 | 0.004 | 0.281 | 0.110 |
| Quillaja saponaria saponin | 1:0.25 | 0.000 | 0.054 | 0.057 |
| | 1:1 | 0.003 | 0.136 | 0.082 |
| | 1:4 | 0.007 | 0.250 | 0.532 |
| Tibulus terrestris saponin | 1:0.25 | 0.000 | 0.081 | 0.068 |
| | 1:1 | 0.002 | 0.149 | 0.087 |
| | 1:4 | 0.009 | 0.246 | 0.154 |

Referring to Table 2 and Table 1, it was found that in the case that tea saponin, American ginseng saponin, Panax ginseng saponin (leaf), Panax ginseng saponin (root), and soy saponin were added, increase effect of solubilities of PSNPPC (that is, solubility with respect to water) were excellent compared to those in cases of other additives except the case of Tween^{®} 80. Tween^{®} 80 is effective when it comes to improving solubility, however, does not have a physiological function.

### Example 1: Preparation of tablet

A tablet was prepared using resveratrol, Panax ginseng saponin (root), and so on. The prepared tablet had a composition according to Table 3 below.

**[Table 3]**

| Ingredients (manufacturer, trade name) | Amount (mg) |
|---|---|
| Resveratrol (NATUREX) | 200 |
| Panax ginseng saponin (root) (Hong-ziu Biotech) | 400 |
| Microcrystalline cellulose (FMC, Avicel PH102) | 145 |
| Crospovidone (BASF, Kollidon CL) | 40 |
| Colloidal silicon dioxide (HDK N20, Wacker Chemie AG) | 5 |
| Magnesium stearate (Won-Poong Pharm) | 10 |
| Total weight | 800 |

### Example 2: Preparation of capsule

A capsule was prepared using resveratrol, Panax ginseng saponin (root) and so on. The prepared capsule had a composition according to Table 4 below.

**[Table 4]**

| Ingredients (manufacturer, trade name) | Amount (mg) |
|---|---|
| Resveratrol (NATUREX) | 200 |
| Panax ginseng saponin (root) (Hong-ziu Biotech) | 400 |
| Lactose (Sheffiled, Lactose Anhydrous 60M) | 150 |
| Starch (National Starch) | 130 |
| Colloidal silicon dioxide (Wacker Chemie AG, HDK N20) | 10 |
| Magnesium stearate (Won-Poong Pharm) | 10 |
| Total weight | 900 |

### Example 3: Preparation of liquid formulation

A capsule was prepared using resveratrol, Panax ginseng saponin (root) and so on. The preapred capsule had a composition according to Table 4 below.

**[Table 5]**

| Ingredients (manufacturer, trade name) | Amount (mg) |
|---|---|
| Resveratrol (NATUREX) | 40 |
| Panax ginseng saponin (root) (Hong-ziu Biotech) | 160 |
| Powdered sugar (Southern Home, Confectioners-sugar), | 400 |
| Isomerized sugar (Fructofin, Danisco) | 400 |
| Stevioside 50 wt%(Stevian Biotechnology Corporation) | 0.23 |
| Lemon flavor (Bolak) | 0.05 |
| Purified water | 20,000 |
| Total amount | 21,000.28 |

### Example 4: Preparation of healthy beverage

A healthy beverage was prepared using resveratrol, Panax ginseng saponin (root) and so on. The prepared healthy beverage had a composition according to Table 6 below.

**[Table 6]**

| Ingredients (manufacturer, trade name) | Amount (mg) |
|---|---|
| Resveratrol (NATUREX) | 100 |
| Panax ginseng saponin (root) (Hong-ziu Biotech) | 400 |
| Citric acid (Jiali Bio Group (Qingdao) Limited) | 100 |
| Oligosaccharide (S.W.C.T, isomaltooligosaccharide) | 2 |
| Taurine (S.W.C.T) | 500 |
| Purified water | 100,000 |
| Total amount | 101,102 |

### Example 5: Preparation of functional healthy food

A functional healthy food was prepared using resveratrol, Panax ginseng saponin (root) and so on. The prepared functional healthy food had a composition according to Table 7 below.

**[Table 7]**

| Ingredients (manufacturer, trade name) | Amount (mg) |
|---|---|
| Resveratrol (NATUREX) | 100 |
| Panax ginseng saponin (root) (Hong-ziu Biotech) | 200 |
| Vitamin A (Won-Poong Pharm) | 500 |
| DL-alpha tocopherol (Won-Poong Pharm) | 20 |
| Vitamin B1 nitrate (Won-Poong Pharm) | 1.5 |
| Vitamin 2 (Won-Poong Pharm) | 1.5 |
| Pyridoxine hydrochloride (Won-Poong Pharm) | 2 |
| Vitamin B12(Won-Poong Pharm) | 2 |
| Biotin (Won-Poong Pharm) | 0.1 |
| Nicotinamide (Won-Poong Pharm) | 5 |
| Folic acid (Won-Poong Pharm) | 0.1 |
| Calcium pantothenate (Won-Poong Pharm) | 5 |
| Vitamin C (Won-Poong Pharm) | 50 |
| calcium glycerophosphate (calcium amount: 4.76 wt%) (Won-Poong Pharm) | 25 |
| Iron sulfate (iron amount: 5.0 wt%)(Won-Poong Pharm) | 15.45 |
| Zinc sulfate (zinc amount: 1.0 wt%) (Won-Poong Pharm) | 2.74 |
| Copper sulfate (copper amount: 0.5 wt%) (Won-Poong Pharm) | 1.256 |
| Manganese sulfate (manganese amount: 0.58 wt%) (Won-Poong Pharm) | 1.54 |
| Gelled starch (Colorcon, Starch 1500) | 46.814 |
| Colloidal silicon dioxide (Won-Poong Pharm) | 10 |
| Magnesium steatrate (Won-Poong Pharm) | 10 |
| Total amount | 1000 |

While the inventive concept has been particularly shown and described with reference to exemplary embodiments thereof, it will be understood that various changes in form and details may be made therein without departing from the spirit and scope of the following claims.

## Claims

1. A composition comprising a natural polyphenolic compound, the compound comprising a poorly water-soluble natural polyphenolic compound (PSNPPC) and saponin, and having water dispersibility or water solubility.

2. The composition of claim 1, wherein the PSNPPC has a solubility of 0.004 to 0.087.

3. The composition of claim 2, wherein the PSNPPC comprises curcumin, silymarin, resveratrol, or a combination thereof.

4. The composition of claim 1, wherein the saponin comprises tea saponin, American ginseng saponin, Panax ginseng saponin (leaf), Panax ginseng saponin (root), soy saponin, or a combination thereof.

5. The composition of claim 1, wherein, when dissolving the composition, a solubility of the PSNPPC is in a range of 0.006 to 1.087.

6. The composition of claim 5, wherein an amount of the saponin is in a range of 25 to 400 parts by weight based on 100 parts by weight of the PSNPPC.

7. An orally ingestible composition, the composition comprising the composition of any one of claims 1 to 6.

8. The composition of claim 7, wherein the composition is a functional healthy food or a medicine in a form of a tablet, a capsule, a liquid formulation, or a beverage.

9. The composition of claim 7, wherein an amount of the saponin in the composition is in a range of 0.4 to 50 wt%.
